# EUROPÄISCHE PATENTSCHRIFT

(11) **EP 1 527 790 B1**
(45) Veröffentlichungstag und Bekanntmachung des Hinweises auf die Patenterteilung: **13.08.2008**
(21) Anmeldenummer: 03405769.5
(22) Anmeldetag: 27.10.2003
(51) Int. Cl.: A61L 27/04, A61L 27/30, A61L 27/10

(54) **Implantat mit einer keramischen Beschichtung**
Implant with a ceramic coating
Implant avec un revêtement céramique

(43) Veröffentlichungstag der Anmeldung: 04.05.2005
(73) Patentinhaber: Straumann Holding AG, 4002 Basel (CH)
(72) Erfinder: Breitenstein, Michael, 4493 Wenslingen (CH); Wieland, Marco, 4054 Basel (CH)
(74) Vertreter: Schaad, Balass, Menzl & Partner AG

(56) Entgegenhaltungen:
- EP-A- 0 534 078
- EP-A- 0 534 978
- FR-A- 2 788 963
- US-A- 4 746 532
- US-A- 4 846 837
- US-A- 5 478 237

## Beschreibung

Die Erfindung betrifft Implantate mit einer keramischen Beschichtung sowie ein Verfahren zum keramischen Beschichten eines Implantats.

In der Praxis haben sich insbesondere in der Dental-Implantologie Implantate bewährt, bei welchen die Knochen-Kontaktfläche aus Metallen, insbesondere Titan oder Titanlegierungen gebildet ist. Insbesondere im hydroxylierten und hydrophilen Zustand kann mit solchen Werkstoffen eine hervorragende Osteo-Integration bewirkt werden (siehe z.B. WO 00/44305).

Im Bereich des Weichgewebes hingegen sind derartige Werkstoffe mit Nachteilen behaftet: Zum einen schimmert das metallischdunkle Implantat durch das Weichgewebe, wodurch der optische Eindruck einer solchen Versorgung getrübt sein kann. Darüber hinaus ist beschrieben, dass mit anderen Werkstoffen, bspw. Keramiken, im Bereich des Weichgewebes eine verbesserte Gewebereaktion bewirkt werden kann (siehe z.B. das deutsche Gebrauchsmuster DE-U1-298 20 971).

Es ist daher aus einer Vielzahl von Dokumenten bekannt, ein Metallimplantat mit einer Keramikmanschette auszustatten, die im Bereich der Weichteil-Kontaktfläche angeordnet ist, siehe z.B. die Dokumente DE-U1-298 20 971, US 5,152,687, FR-B3-2 788 963, GB-A-2 139 095. Derartige Keramikmanschetten werden beispielsweise an dem Implantat aufgesintert oder verklebt. Hierdurch ergeben sich jedoch mehrere bislang nicht gelöste Probleme. Einerseits ist durch die Verwendung einer Keramikmanschette als separates Bauteil ein Mikrospalt nahezu unvermeidbar, wodurch insbesondere bakterielle Kontaminationen hervorgerufen werden können.

Beim Aufsintern einer solchen Manschette hingegen müssen in der Regel derart hohe Temperaturen verwendet werden, dass der metallische Implantat-Grundkörper zumindest teilweise oxidiert. Dies wirkt sich jedoch nachteilig auf die Osteo-Integration aus. Zudem können durch unterschiedliche Wärmeausdehnungskoeffizienten der beteiligten Materialien insbesondere beim Abkühlen Mikrospannungen auftreten, welche zu Haarrissen und Sprüngen führen können.

Es ist z.B. aus dem Dokument EP-B1-0 211 676 bekannt, Titanimplante mit einer Keramikbeschichtung des gesamten Implantatkörpers zu versehen. Ebenso wurde vorgeschlagen, die Knochen-Kontaktfläche eines Implantats mit einer biokompatibelen Beschichtung bspw. aus Hydroxyapatit oder Kalziumphosphat zu versehen (z.B. US 5,478,237). Hierbei stehen jedoch die bewährten, vorteilhaften Eigenschaften des Titans für die Osteo-Integration nicht mehr zur Verfügung.

Zur Beschichtung eines Substrats sind eine Vielzahl von Verfahren bekannt:
Beschichtungen können mittels der Verfahren CVD oder PVD (chemical vapor deposition bzw. physical vapor deposition) aufgebracht werden. Nachteilig hierbei ist eine im allgemeinen nicht optimale Adhäsion an der Oberfläche sowie ggf. Brüchigkeit der Beschichtung. Zudem ist die Farbe einer derartig erzielbaren Beschichtung durch deren Dicke im allgemeinen nicht im gewünschten Masse beeinflussbar.

Weiterhin sind Beschichtungen mit Silikatglass und mit Silikatglass modifizierten Keramiken bekannt, z.B. aus dem Dokument WO 01/74730. Nachteilig hierbei ist wiederum die Möglichkeit des Auftretens von Mikrospannungen durch den Brennprozess bei hohen Temperaturen sowie die zumindest teilweise Oxidation der Titanoberfläche.

Es ist darüber hinaus bekannt, Metalle wie Aluminium, Titan oder Tantal mittels anodischer Oxidation unter Funkenentladung zu beschichten: Das Dokument DE-C1-43 03 575 beschreibt die Herstellung von apatitbeschichteten Implantaten. Das Dokument DD-A1-246 028 beschreibt ein Metallimplantat, welches im Knochen-Kontaktbereich mit einer Kalziumphosphatkeramik beschichtet ist. Auch hierbei stehen jedoch die bewährten, vorteilhaften Eigenschaften insbesondere des Titans für die Osteo-Integration nicht mehr zur Verfügung.

Es ist daher eine Aufgabe der Erfindung, die Nachteile des bekannten zu vermeiden, insbesondere also ein Implantat zu schaffen, welches über die bewährten und vorteilhaften Eigenschaften bekannter Implantate insbesondere aus Titan und Titanlegierungen bezüglich der Osteo-Integration verfügt. Darüber hinaus ist es eine zusätzliche Aufgabe der Erfindung, ein Implantat bereitzustellen, welches eine verbesserte Verträglichkeit besitzt sowie das Durchschimmern von Metall im Weichgewebebereich verhindert. Ein solches Implantat soll zudem kostengünstig und mit bewährten Verfahren herstellbar sein.

Erfindungsgemäss werden diese Aufgaben mit einem Implantat sowie einem Verfahren zum keramischen Beschichten gemäss der unabhängigen Patentansprüche gelöst.

Ein erfindungsgemässes Implantat, insbesondere ein Dental-Implantat besitzt einen Grundkörper mit mindestens einer Knochen-Kontaktfläche und mindestens einer Weichgewebe-Kontaktfläche, wobei die Weichgewebe-Kontaktfläche und/oder ein Übergangsbereich von Knochen-Kontaktfläche zu Weichgewebe-Kontaktfläche zumindest teilweise eine keramische Beschichtung aufweist. Vorzugsweise ist die gesamte Knochen-Kontaktfläche nicht mit einer keramischen Beschichtung versehen. Die Knochen-Kontaktfläche weist jedoch zumindest Bereiche auf, welche nicht mit einer keramischen Beschichtung versehen sind. Als Übergangsbereich von Knochen-Kontaktfläche zu Weichgewebe-Kontaktfläche werden hier und im folgenden insbesondere jegliche Oberflächen des Implantats verstanden, welche im implantierten Zustand ggf. sowohl mit umliegendem Weichgewebe als auch dem Knochen in Kontakt kommen können. Durch ein derartiges Implantat stehen die bekannt vorteilhaften und vielfach bewährten Osteo-Integrationseigenschaften bspw. des Titans im Bereich der Knochen-Kontaktfläche weiterhin zur Verfügung. Titan bzw. eine Titanlegierung ist ein bevorzugtes Material des Grundkörpers eines erfindungsgemässen Implantats, die Erfindung ist jedoch nicht hierauf zu beschränken. Zumindest der Grundkörper des Implantats kann aus jeglichem gewebeverträglichen Material, insbesondere einem gewebeverträglichen Metall oder einer Legierung eines solchen Metalls bestehen, bspw. also neben Titan auch Tantal, Zirkonium und Hafnium, sowie Legierungen dieser Metalle, insbesondere jeweils mit Zusätzen von Niob und/oder Tantal und/oder Hafnium. Im Bereich der Weichgewebe-Kontaktfläche ist durch die keramische Beschichtung eine verbesserte Gewebeverträglichkeit sowie eine verbesserte Ästhetik bewirkt. Die meist weisse oder weiss-gräuliche keramische Beschichtung schimmert nicht bzw. nur unwesentlich durch das Weichgewebe hindurch; zudem ist die keramische Beschichtung auch leicht durch geeignete Beimengungen, welche dem Fachmann bekannt sind, farblich anpassbar, so dass auch Farbnuancen (bspw. zahnfleischfarben, rosa, etc.) ohne weiteres erzielbar sind. Unter keramischen Beschichtungen werden hier und im folgenden insbesondere feine, dichte sonderkeramische Werkstoffe verstanden, insbesondere Oxidkeramiken.

In einer bevorzugten Ausführungsform ist die nicht mit einer keramischen Beschichtung versehene Knochen-Kontaktfläche derart behandelt, dass eine verbesserte Osteo-Integration bewirkt ist. Derartige Behandlungen sind bspw. in den Dokumenten WO 00/44305 und WO 02/07792 beschrieben, deren Offenbarung hiermit durch Referenz eingeschlossen ist. Insbesondere betreffen bevorzugte Ausführungsformen einer solchen Oberflächenbehandlung solche Behandlungen, welche in einer aufgerauhten Oberfläche in hydroxyliertem und hydrophilen Zustand resultieren. Hierbei sind insbesondere maximale Rauhtiefen Rₘₐₓ von 0.1 µm bis 50 µm nach DIN 4768 bevorzugt (Profilhöhe innerhalb der Mess-Strecke), welche u.a. durch mechanische Behandlung (bspw. Sandstrahlen, Kugelstrahlen, etc.) und/oder chemische Behandlung (bspw. Ätzung) oder auch elektrochemische Verfahren bewirkt sein können.

In einer besonders bevorzugten Ausführungsform der Erfindung ist die keramische Beschichtung durch ein Verfahren der anodischen Oxidation, insbesondere unter Funkenentladung, aufgebracht. Ein Verfahren zur anodischen Oxidation unter Funkenentladung (ANOF) ist u.a. in den Dokumenten DE-C1-43 03 575 und DD-A1-246 028 sowie in Z. Klin. Med. 41 (1986), Heft 3, S. 219-222 beschrieben; die Offenbarung dieser Dokumente ist hiermit durch Referenz eingeschlossen. Es handelt sich hierbei um ein Verfahren zur Schichtbildung, bei welchem eine Oxidschicht unter Plasmabedingungen erschmolzen wird. Besonders bevorzugt handelt es sich bei einer solchen Oxidschicht um eine im wesentlichen arteigene Oxidschicht (z.B. Grundkörper des Implantats aus Titan: Titanoxid-Beschichtung, etc.), die Erfindung ist jedoch nicht auf solche Ausführungsformen zu beschränken. Mittels anodischer Oxidation unter Funkenentladung können ohne weiteres besonders hohe Haftfestigkeiten der aufgebrachten Beschichtung von > 8 Mpa erzielt werden, wodurch derart erzielbare Beschichtungen bezüglich der Haltbarkeit bevorzugt sind gegenüber anderweitig erzielbaren Beschichtungen. Zudem ist kein abschliessendes Aufbrennen der Beschichtung erforderlich, wodurch die insbesondere aus Titan oder einer Titanliegierung bestehende Knochen-Kontaktfläche in Mitleidenschaft gezogen, insbesondere zumindest teilweise oxidiert würde. Besonders vorteilhafterweise wird zur Verbesserung der Haftfestigkeit der keramischen Beschichtung der zur Beschichtung vorgesehene Bereich des Grundkörpers des Implantats vorgängig aufgerauht, bspw. durch Ätzung.

Besonders bevorzugt beinhaltet die keramische Beschichtung eine Titanoxid-Keramik und/oder Zirkonoxid-Keramik und/oder eine Aluminiumoxid-Keramik. Derartige Beschichtungen kommen besonders vorteilhaft als arteigene Beschichtungen zum Einsatz, bspw. wird also bevorzugt der Grundkörper eines Implantats aus Titan mit einer Titanoxidkeramik versehen. Selbstverständlich sind jedoch auch andersartige keramische Beschichtungen und auch Mischungen möglich, um bspw. besondere Farbnuancen oder bestimmte Rauhtiefen zu bewirken.
Gemäss einer weiteren, besonders bevorzugten Ausführungsform beträgt die Dicke der keramischen Beschichtung 1 nm bis 1 mm, vorzugsweise 200 nm bis 100 µm. Die Dicke der keramischen Beschichtung ist hierbei insbesondere derart gewählt, dass ein Durchscheinen des metallischen Materials des Grundkörpers des Implantats durch das Weichgewebe verhindert werden kann.

In einer weiteren Ausgestaltung der Erfindung ist die chemische Zusammensetzung und/oder eine physikalische Eigenschaft der keramischen Beschichtung entlang der Dicke der Schicht insbesondere kontinuierlich variiert. Insbesondere ist es z.B. möglich, durch Beimischungen während des Abscheidens der Beschichtung insbesondere durch anodische Oxidation unter Funkenentlandung eine Variation der Farbe entlang der Dicke der Schicht eine besondere Farbtiefe zu bewirken. Für besondere Anwendungszwecke kann aber auch graduell entlang der Dicke der keramischen Beschichtung eine Komponente beigemischt werden, welche bspw. die Weichgewebeverträglichkeit weiter verbessert, bspw. Calcium, Phosphate, Collagen, Fibrin, Aminosäuren, aktive Zahnschmelz-Substanzen wie bspw. EMDOGAIN®, etc.. Zudem ist es denkbar, eine physikalische Eigenschaft der keramischen Beschichtung entlang der Dicke der Beschichtung zu variieren, insbesondere die Dichte und/oder die Porengrösse, wobei die Porengrösse vorzugsweise aufgrund der potentiellen Anhaftung von Bakterien und sonstigen Verunreinigungen möglichst klein gewählt ist. Hierdurch kann selektiv ein Einfluss auf die Oberflächenbeschaffenheit ausgeübt werden, wodurch die Interaktion mit umliegendem Gewebe beeinflusst werden kann.

Gemäss einer weiteren Ausführungsform der Erfindung weist die keramische Beschichtung eine Rauhtiefe Rₘₐₓ nach DIN 4768 von 100 nm bis 100 µm, vorzugsweise von 100 nm bis 50 µm, besonders bevorzugt von 100 nm bis 5 µm auf. Eine derartige Rauhigkeit der Oberfläche kann sowohl bereits während des Auftragens, insbesondere durch anodische Oxidation mittels Funkenentladung, als auch durch nachträgliche, abhebende, insbesondere mechanische Behandlung aufgebracht sein. Die Rauhigkeit ist hierbei derart gewählt, dass eine vorteilhafte Interaktion mit umliegendem Gewebe bewirkt ist, jedoch kein hygienisch bedenkliches Milieu für Bakterien durch zu grobe Strukturierung, insbesondere eine grobe, offene Porösität, geschaffen ist. Selbstverständlich sind auch nach dem Auftragen Zusatzbehandlungen der keramischen Beschichtung wie bspw. Ätzen oder Sandstrahlen möglich, insbesondere um die Oberflächenbeschaffenheit zu modifizieren.

Gemäss weiterer vorteilhafter Ausführungsformen der Erfindungen kann insbesondere ein Übergangsbereich von Knochen-Kontaktfläche zu Weichgewebe-Kontaktfläche auf verschieden Weise ausgebildet sein.
So ist es möglich und vorteilhaft, dass die Dicke der keramischen Beschichtung gegen die Knochen-Kontaktfläche hin insbesondere kontinuierlich abnimmt. Hierbei ist es selbstverständlich möglich, dass die Dicke der Schicht über ihre gesamte Ausdehnung oder auch lediglich in einem Übergangsbereich von Knochen-Kontaktfläche zu Weichgewebe-Kontaktfläche insbesondere kontinuierlich abnimmt. Die Variation der Dicke kann bereits während des Auftragens bzw. Abscheidens der keramischen Beschichtung als auch nachträglich durch abtragende, insbesondere mechanische Behandlung bewirkt sein.
In einer weiteren vorteilhaften Ausgestaltungsform reicht die keramische Beschichtung bis zu einem Vorsprung insbesondere in einem Übergangsbereich von Knochen-Kontaktfläche und Weichgewebe-Kontaktfläche herab, welcher Vorsprung durch die keramische Beschichtung ausgeglichen ist. Hierbei ist es nicht notwendig, die Dicke der keramischen Beschichtung in einem Übergangsbereich von Knochen-Kontaktfläche und Weichgewebe-Kontaktfläche in der Dicke zu reduzieren, um keine hervorstehenden Abschlusskanten zu erhalten, wodurch die Herstellung eines solchen Implantats erheblich vereinfacht ist.

Gemäss einer besonders bevorzugten Ausführungsform eines erfindungsgemässen Implantats ist der der Knochen-Kontaktfläche zugewandte Rand der keramischen Beschichtung entlang des Umfangs des Implantats kurvenförmig mit mindestens einer, vorzugsweise zwei Erhebungen und einer, vorzugsweise zwei Senken gebildet, so dass die natürliche Knochenform durch diesen Rand nachvollzogen ist. Hierdurch kann sichergestellt werden, dass idealerweise sämtliche Weichgewebe-Kontaktflächen mit der keramischen Beschichtung versehen sind, und insbesondere nicht in einem Übergangsbereich von Knochen-Kontaktfläche zu Weichgewebe-Kontaktfläche unbeschichtete Flächen vorhanden sind, welche nicht in Kontakt mit dem Knochen stehen. Durch eine derartige Form des Randes der keramischen Beschichtung kann wirkungsvoll ein Durchscheinen der Farbe des Grundkörpers des Implantats im implantierten Zustand durch das Weichgewebe verhindert werden. Die Ausgestaltung des Randes kann hierbei sowohl für die meisten Knochenformen universell passend als auch individuell, also für jedes Implantat einzeln erfolgen. Insbesondere ist es möglich, mittels CAD/CAM die zu beschichtende Fläche des Implantats über die Knochenform des Patienten exakt zu erfassen und das Implantat entsprechend mit der keramischen Beschichtung zu versehen. Auch ist es möglich, durch abtragende Bearbeitung eine im Überschuss aufgetragene, keramische Beschichtung den individuellen Bedingungen des Knochens anzupassen.

Die Erfindung betrifft darüber hinaus ein Verfahren zum insbesondere keramischen Beschichten eines Implantats, wobei eine keramische Beschichtung lediglich auf die Weichgewebe-Kontaktfläche und/oder einen Übergangsbereich von Knochen-Kontaktfläche und Weichgewebe-Kontaktfläche eines osteophilen Implantats aufgebracht wird. Vorzugsweise wird hierfür ein Verfahren der anodischen Oxidation, insbesondere unter Funkenentladung, angewendet.

In einer bevorzugten Ausführungsform werden Bereiche des Implantats derart zumindest temporär abgedeckt, dass sie einer keramischen Beschichtung nicht zugänglich sind. Hierfür werden vorzugsweise elastomere Abdeckungen verwendet, bspw. das Fluoroelastomer Viton® von DuPont Dow Elastomers. Hierdurch kann mit einfachen Mitteln eine präzise Beschichtung lediglich des gewünschten Bereichs des Grundkörpers des Implantats bewirkt werden.

Gemäss einer weiteren bevorzugten Ausführungsform wird die Oberfläche der keramischen Beschichtung zumindest teilweise durch physikalische und/oder chemische Prozesse aufgerauht. Ein solches Aufrauhen der Oberfläche kann insbesondere erfolgen durch mechanische Behandlung (bspw. Sandstrahlen, Kugelstrahlen, etc.) und/oder chemische Behandlung (bspw. Ätzung) oder auch elektochemische Verfahren. Eine solche nachträglich erfolgende Aufrauhung kann zusätzlich zu der schon beispielsweise während des Beschichtens mittels anodischer Oxidation unter Funkenentladung erzielbaren und in gewissem Rahmen steuerbaren Rauhigkeit angewendet werden.

Die Erfindung wird nachfolgend anhand von Figuren und Ausführungsbeispielen erläutert, ohne dass die Erfindung auf die gezeigten Ausführungsformen zu beschränken wäre. Es zeigen:
- Fig. 1:: Verschiedene Flächen eines Implantats;
- Fig. 2a,b:: Ausgestaltungen der keramischen Beschichtung eines Implantats;
- Fig. 3:: keramische Beschichtung, die natürliche Knochenform nachvollziehend.

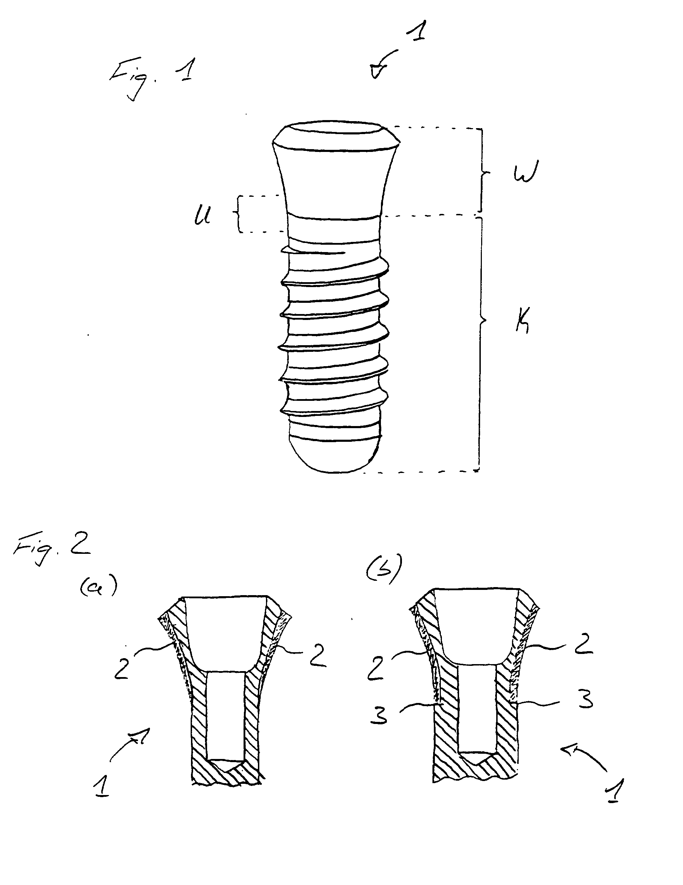

Figur 1 zeigt die verschiedenen Bereiche eines Implantats 1. Ein solches Implantat 1 ist bevorzugt hergestellt aus einem gewebeverträglichen Metall oder einer Legierung eines solchen Metalls, insbesondere aus Titan oder einer Titanlegierung. Ein Implantat 1 ist untergliedert in mindestens eine Knochen-Kontaktfläche K und eine Weichgewebe-Kontaktfläche W. Im Grenzbereich dieser Flächen befindet sich ein Übergangsbereich U von Knochen-Kontaktfläche K zu Weichgewebe-Kontaktfläche W, welcher beiden vorgenannten Flächen zuzuordnen ist. Ob sich dieser Bereich im implantierten Zustand im Knochen oder im Weichgewebe befindet, hängt von einer Vielzahl Faktoren ab wie z.B. der Einschraubtiefe, der Gewebereaktion etc.. Im Bereich der Knochen-Kontaktfläche K und des Übergangsbereich U kann die Oberfläche derart behandelt sein, dass die Osteointegrationseigenschaften des Implantats 1 verbessert sind. Im Falle von Implantaten 1 aus Titan ist hierbei eine aufgerauhte, hydroxylierte und hydrophile Oberfläche bevorzugt. Selbstverständlich ist die Erfindung nicht auf die gezeigte Implantatform, insbesondere nicht auf die gezeigte Schnittstelle zur Befestigung eines Aufbauteils zu beschränken; eine keramische Beschichtung 2 im Sinne der vorliegenden Erfindung ist mit jedem Implantat 1 zweckmässig und realisierbar, solange ein definierter Bereich des Implantats 1 im Weichgewebe anzuordnen ist, das Implantat 1 also über eine Weichgewebe-Kontaktfläche verfügt.

Figur 2 zeigt ein Implantat 1 gemäss Figur 1 mit einer keramischen Beschichtung 2. Die keramische Beschichtung 2 ist im Bereich der Weichgewebe-Kontaktfläche W und des Übergangsbereich U von Knochen-Kontaktfläche K zu Weichgewebe-Kontaktfläche W angeordnet. In Figur 2a ist ein Ausführungsbeispiel gezeigt, in welchem die Dicke der keramischen Beschichtung 2 gegen die Knochen-Kontaktfläche K hin kontinuierlich abnimmt. Selbstverständlich ist es erfindungsgemäss jedoch bspw. auch möglich, dass die Dikke der keramischen Schicht lediglich in einem der Knochen-Kontaktfläche benachbarten Bereich, insbesondere dem Übergangsbereich U, insbesondere kontinuierlich abnimmt. Eine derartige Variation der Dicke der Schicht kann bereits beim Aufbringen der keramischen Beschichtung realisiert werden, aber auch nachträglich durch insbesondere mechanische Behandlung bewirkt werden. Figur 2b zeigt eine Ausführungsform, bei welcher die keramische Beschichtung 2 bis zu einem Vorsprung 3 des Implantats insbesondere in einem Übergangsbereich U des Implantats reicht, wobei der Vorsprung 3 durch die keramische Beschichtung 2 ausgeglichen ist. Durch eine solche Ausgestaltung eines erfindungsgemässen Implantats 1 ist eine besonders einfache Herstellbarkeit gewährleistet, da weder während der Herstellung noch nachträglich die Dicke der keramischen Beschichtung 2 angepasst werden muss. Im Falle des Aufbringens einer keramischen Beschichtung 2 durch ein Verfahren der anodischen Oxidation unter Funkenentladung kann die Dicke der Beschichtung 2 durch geeignete Wahl der Abscheidungsdauer derart beeinflusst werden, dass der Vorsprung 3 gerade ausgeglichen ist. Der in den Figuren 2a und 2b gezeigte, abgeschrägte obere Abschlussbereich des Implantats 1 ist in dem Ausführungsbeispiel nicht mit einer keramischen Beschichtung 2 versehen; dieser Bereich bildet die Schnittstelle zu einem nicht im Detail gezeigten Aufbauelement und ist daher vorteilhaft von der keramischen Beschichtung 2 ausgespart.

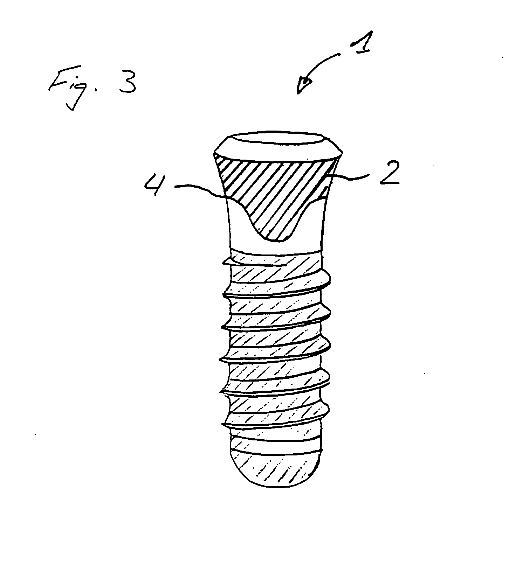

Figur 3 zeigt eine besonders bevorzugte Ausführungsform eines erfindungsgemäss mit einer keramischen Beschichtung 2 versehenen Implantats 1. Hierbei ist der Rand 4 der keramischen Beschichtung 2 entlang des Umfangs des Implantats 1 kurvenförmig mit mindestens einer Erhebung und einer Senke ausgebildet, so dass im implantierten Zustand die natürliche Knochenform durch diesen Rand 4 nachvollzogen ist. Hierdurch kann sichergestellt werden, dass idealerweise sämtliche Weichgewebe-Kontaktflächen W mit der keramischen Beschichtung 2 versehen sind, und insbesondere nicht in einem Übergangsbereich U von Knochen-Kontaktfläche K zu Weichgewebe-Kontaktfläche W unbeschichtete Flächen vorhanden sind, welche nicht in Kontakt mit dem Knochen stehen. Insbesondere im Bereich der Knochen-Kontaktfläche K kann wiederum eine Oberflächenbehandlung zur verbesserten Osteointegration vorgesehen sein, insbesondere eine aufgerauhte, hydroxylierte und hydrophile Oberfläche. Hierbei ist eine solche Oberflächenbehandlung nicht notwendigerweise bis an die keramische Beschichtung 2 heranreichend ausgebildet.

## Patentansprüche

1. Implantat (1) mit einem Grundkörper mit mindestens einer Knochen-Kontaktfläche (K) und mindestens einer Weichgewebe-Kontaktfläche (W), **dadurch gekennzeichnet, dass** die Weichgewebe-Kontaktfläche (W) und/oder ein Übergangsbereich (U) von Knochen-Kontaktfläche (K) zu Weichgewebe-Kontaktfläche (W) zumindest teilweise eine keramische Beschichtung (2) aufweisen, die durch ein Verfahren der anodischen Oxidation aufgebracht ist, und dass die Knochen-Kontaktfläche (K) Bereiche aufweist, welche nicht mit einer keramischen Beschichtung (2) versehen sind.

2. Implantat (1) nach Anspruch 1, **dadurch gekennzeichnet, dass** der Grundkörper zumindest teilweise aus einem gewebeverträglichen Metall oder einer Legierung eines solchen Metalls besteht.

3. Implantat (1) nach Anspruch 2, **dadurch gekennzeichnet, dass** der Grundkörper zumindest teilweise aus Titan oder einer Titanlegierung besteht.

4. Implantat (1) nach einem der Ansprüche 1 bis 3, **dadurch gekennzeichnet, dass** die keramische Beschichtung (2) durch ein Verfahren der anodischen Oxidation unter Funkenentladung aufgebracht ist.

5. Implantat (1) nach einem der Ansprüche 1 bis 4, **dadurch gekennzeichnet, dass** die keramische Beschichtung (2) eine Titanoxid-Keramik und/oder Zirkonoxid-Keramik beinhaltet.

6. Implantat (1) nach einem der Ansprüche 1 bis 5, **dadurch gekennzeichnet, dass** die Dicke der keramischen Beschichtung (2) 1 nm bis 1 mm, vorzugsweise 200 nm bis 100 µm beträgt.

7. Implantat (1) nach Anspruch 6, **dadurch gekennzeichnet, dass** die chemische Zusammensetzung und/oder eine physikalische Eigenschaft der keramischen Beschichtung (2) entlang der Dicke der Schicht insbesondere kontinuierlich variiert.

8. Implantat (1) nach einem der Ansprüche 1 bis 7, **dadurch gekennzeichnet, dass** die keramische Beschichtung (2) eine Rauhtiefe Rₘₐₓ nach DIN 4768 von 100 nm bis 100 µm, vorzugsweise von 100 nm bis 50 µm, besonders bevorzugt von 100 nm bis 5 µm aufweist.

9. Implantat (1) nach einem der Ansprüche 1 bis 8, **dadurch gekennzeichnet, dass** die Dicke der keramischen Beschichtung (2) gegen die Knochen-Kontaktfläche (K) hin insbesondere kontinuierlich abnimmt.

10. Implantat (1) nach einem der Ansprüche 1 bis 8, **dadurch gekennzeichnet, dass** die keramische Beschichtung (2) bis zu einem Vorsprung (3) insbesondere in einem Übergangsbereich (U) von Knochen-Kontaktfläche (K) und Weichgewebe-Kontaktfläche (W) reicht, welcher Vorsprung (3) durch die keramische Beschichtung (2) ausgeglichen ist.

11. Implantat (1) nach einem der Ansprüche 1 bis 10, **dadurch gekennzeichnet, dass** der Rand (4) der keramischen Beschichtung (2) entlang des Umfangs des Implantats (1) kurvenförmig mit mindestens einer Erhebung und einer Senke gebildet ist, so dass die natürliche Knochenform durch diesen Rand (4) nachvollzogen ist.

12. Verfahren zum insbesondere keramischen Beschichten eines Implantats, wobei eine keramische Beschichtung (2) durch ein Verfahren der anodischen Oxidation auf die Weichgewebe-Kontaktfläche (W) und/oder einen Übergangsbereich (U) von Knochen-Kontaktfläche (K) und Weichgewebe-Kontaktfläche (W) eines Implantats (1) aufgebracht wird.

13. Verfahren nach Anspruch 12, wobei die keramische Beschichtung (2) mittels anodischer Oxidation, unter Funkenentladung aufgebracht wird.

14. Verfahren nach einem der Ansprüche 12 bis 13, **dadurch gekennzeichnet, dass** Bereiche des Implantats (1) derart zumindest temporär abgedeckt werden, dass sie einer keramischen Beschichtung nicht zugänglich sind.

15. Verfahren nach einem der Ansprüche 12 bis 14, **dadurch gekennzeichnet, dass** die Oberfläche der keramischen Beschichtung (2) zumindest teilweise durch physikalische und/oder chemische Prozesse aufgerauht wird.

## Claims

1. Implant (1) with a base body having at least one bone contact surface (K) and at least one soft tissue contact surface (W), **characterized in that** the soft tissue contact surface (W) and/or a transition area (U) from bone contact surface (K) to soft tissue contact surface (W) have/has at least partially a ceramic coating (2) which is applied by a method of anodic oxidation, and **in that** the bone contact surface (K) has areas not provided with a ceramic coating (2).

2. Implant (1) according to Claim 1, **characterized in that** the base body is made at least partially of a tissue-compatible metal or of an alloy of such a metal.

3. Implant (1) according to Claim 2, **characterized in that** the base body is made at least partially of titanium or of a titanium alloy.

4. Implant (1) according to one of Claims 1 to 3, **characterized in that** the ceramic coating (2) is applied by a method of anodic oxidation under spark discharge.

5. Implant (1) according to one of Claims 1 to 4,
**characterized in that** the ceramic coating (2) contains a titanium oxide ceramic and/or zirconium oxide ceramic.

6. Implant (1) according to one of Claims 1 to 5, **characterized in that** the thickness of the ceramic coating (2) is 1 nm to 1 mm, preferably 200 nm to 100 µm.

7. Implant (1) according to Claim 6, **characterized in that** the chemical composition and/or a physical property of the ceramic coating (2) varies, in particular continuously, along the thickness of the layer.

8. Implant (1) according to one of Claims 1 to 7, **characterized in that** the ceramic coating (2) has a peak-to-valley height Rₘₐₓ, according to DIN 4768, of 100 nm to 100 µm, preferably of 100 nm to 50 µm, particularly preferably of 100 nm to 5 µm.

9. Implant (1) according to one of Claims 1 to 8, **characterized in that** the thickness of the ceramic coating (2) decreases, in particular continuously, towards the bone contact surface (K).

10. Implant (1) according to one of Claims 1 to 8,
**characterized in that** the ceramic coating (2) reaches as far as a projection (3) in particular in a transition area (U) from bone contact surface (K) to soft tissue contact surface (W), which projection (3) is compensated by the ceramic coating (2).

11. Implant (1) according to one of Claims 1 to 10, **characterized in that** the edge (4) of the ceramic coating (2) is formed along the circumference of the implant (1) in a curved shape, with at least one rise and one dip, so that the natural shape of the bone is simulated by this edge (4).

12. Method for in particular ceramic coating of an implant, in which method a ceramic coating (2) is applied by a method of anodic oxidation to the soft tissue contact surface (W) and/or to a transition area (U) from bone contact surface (K) to soft tissue contact surface (W) of an implant (1).

13. Method according to Claim 12, in which the ceramic coating (2) is applied by means of anodic oxidation under spark discharge.

14. Method according to either of Claims 12 and 13, **characterized in that** areas of the implant (1) are at least temporarily covered over so that they are not accessible to a ceramic coating.

15. Method according to one of Claims 12 to 14, **characterized in that** the surface of the ceramic coating (2) is at least partially roughened by physical and/or chemical processes.

## Revendications

1. Implant (1) doté d'un corps de base qui présente au moins une surface (K) de contact avec l'os et au moins une surface (W) de contact avec les tissus mous,
**caractérisé en ce que**
au moins une partie de la surface (W) de contact avec les tissus mous et/ou d'une zone de transition (U) entre la surface (K) de contact avec l'os et la surface (W) de contact avec les tissus mous présente un revêtement céramique (2) qui est appliqué à l'aide d'un procédé d'oxydation anodique
et **en ce que** la surface (K) de contact avec l'os présente des zones qui ne sont pas dotées d'un revêtement céramique (2).

2. Implant (1) selon la revendication 1, **caractérisé en ce qu'**au moins une partie du corps de base est constituée d'un métal compatible avec les tissus ou d'un alliage d'un tel métal.

3. Implant (1) selon la revendication 2, **caractérisé en ce qu'**au moins une partie du corps de base est constituée de titane ou d'un alliage de titane.

4. Implant (1) selon l'une des revendications 1 à 3, **caractérisé en ce que** le revêtement de céramique (2) est appliqué par un procédé d'oxydation anodique sous décharge corona.

5. Implant (1) selon l'une des revendications 1 à 4,
**caractérisé en ce que** le revêtement céramique (2) contient une céramique d'oxyde de titane et/ou une céramique d'oxyde de zirconium.

6. Implant (1) selon l'une des revendications 1 à 5, **caractérisé en ce que** l'épaisseur du revêtement céramique (2) est comprise entre 1 nm et 1 mm et de préférence entre 200 nm et 100 µm.

7. Implant (1) selon la revendication 6, **caractérisé en ce que** la composition chimique et/ou une propriété physique de revêtement céramique (2) varient en particulier de manière continue suivant l'épaisseur de la couche.

8. Implant (1) selon l'une des revendications 1 à 7,
**caractérisé en ce que** le revêtement céramique (2) présente une rugosité d'une profondeur Rₘₐₓ selon DIN 4768 de 100 nm à 100 µm, de préférence de 100 nm à 50 µm et de façon particulièrement préférable de 100 nm à 5 µm.

9. Implant (1) selon l'une des revendications 1 à 8, **caractérisé en ce que** l'épaisseur du revêtement céramique (2) diminue en particulier de manière continue en direction de la surface (K) de contact avec l'os.

10. Implant (1) selon l'une des revendications 1 à 8, **caractérisé en ce qu'**en particulier dans une zone de transition (U) entre la surface (K) de contact avec l'os et la surface (W) de contact avec les tissus mous, le revêtement céramique (2) s'étend jusqu'à une saillie (3), laquelle saillie (3) est compensée par le revêtement céramique (2).

11. Implant (1) selon l'une des revendications 1 à 10, **caractérisé en ce que** le bord (4) du revêtement céramique (2) le long de la périphérie de l'implant (1) est curviligne avec au moins une saillie et un creux, de telle sorte que ce bord (4) épouse la forme naturelle de l'os.

12. Procédé de revêtement en particulier céramique d'un implant, dans lequel un revêtement céramique (2) est appliqué à l'aide d'un procédé d'oxydation anodique sur la surface (W) de contact avec les tissus mous et/ou sur une zone de transition (U) entre la surface (K) de contact avec l'os et la surface (W) de contact avec les tissus mous d'un implant (1).

13. Procédé selon la revendication 12, dans lequel le revêtement céramique (2) est appliqué par oxydation anodique sous décharge corona.

14. Procédé selon l'une des revendications 12 à 13, **caractérisé en ce que** des parties de l'implant (1) sont recouvertes au moins temporairement de manière à ne pas être accessibles au revêtement céramique.

15. Procédé selon l'une des revendications 12 à 14, **caractérisé en ce qu'**au moins une partie de la surface du revêtement céramique (2) est rendue rugueuse par des processus physiques et/ou chimiques.
